# EUROPEAN PATENT SPECIFICATION

(11) **EP 3 434 172 B1**
(45) Date of publication and mention of the grant of the patent: **24.08.2022**
(21) Application number: 17770533.2
(22) Date of filing: 15.03.2017
(51) Int. Cl.: A61B 3/028, A61B 3/032, A61B 3/00

(54) **EYESIGHT EXAMINATION METHOD, EYESIGHT EXAMINATION DEVICE, AND DOWNLOADER SERVER FOR STORING PROGRAM OF EYESIGHT EXAMINATION METHOD**
VERFAHREN ZUR UNTERSUCHUNG DER SEHKRAFT, VORRICHTUNG ZUR UNTERSUCHUNG DER SEHKRAFT UND DOWNLOADER-SERVER ZUM SPEICHERN EINES PROGRAMMS FÜR DAS VERFAHREN ZUR UNTERSUCHUNG DER SEHKRAFT
PROCÉDÉ D'EXAMEN DE LA VUE, DISPOSITIF D'EXAMEN DE LA VUE ET SERVEUR DE TÉLÉCHARGEMENT POUR STOCKER UN PROGRAMME DU PROCÉDÉ D'EXAMEN DE LA VUE

(30) Priority: 23.03.2016 KR 20160034443
(43) Date of publication of application: 30.01.2019
(73) Proprietor: Viewmtechnology Co., Ltd., Gunpo-si, Gyeonggi-do 15809 (KR)
(72) Inventor: KIM, Ki Gon, Seoul 08001 (KR); LEE, Kwang Kyu, Seoul 02834 (KR)
(74) Representative: dompatent von Kreisler Selting Werner - Partnerschaft von Patent- und Rechtsanwälten mbB
(86) International application number: PCT/KR2017/002810
(87) International publication number: WO 2017/164562

(56) References cited:
- WO-A1-2013/059331
- FR-A1- 3 014 673
- JP-A- H0 838 419
- JP-A- H09 224 908
- JP-A- 2015 198 911
- KR-A- 20010 100 713
- KR-B1- 101 451 669
- US-B2- 8 888 288

## Description

### [Technical Field]

The present invention relates to an eyesight examination method, an eyesight examination device and a downloader server for storing a program of the eyesight examination method and, more particularly, to an eyesight examination method, an eyesight examination device and a downloader server for storing a program of the eyesight examination method, in which eyesight examinations can be automatically carried out through simple user inputs and the use of various kinds of optotypes such that accurate eyesight examinations can be carried out uniformly for various users.

### [Background Art]

The eyesight examination is performed in an ophthalmic clinic, an optician's shop, health examination and the like. A typical eyesight examination is carried out through the guide of an optician or a nurse while a subject covers one eye with a cover and sees an eye chart with an uncovered eye at a specific distance away from the chart.

The applicant has recognized a variety of problems in the conventional eyesight examination method and derives a new type of eyesight examination method (Korean Patent Publication No. 10-1451669 B1, published on October 17, 2014, hereinafter, referred to as "patent document 1"). According to patent document 1, an eyesight examination is carried out automatically by displaying two figures represented by lines and receiving user inputs with respect to the displayed two figures.

The invention of patent document 1 receives a user selection (direction selection) according to the recognition of an interval between the lines of inner and outer figures, but it has been found that there are still various drawbacks in the invention of patent document 1 as well.

Both Landolt optotypes or the figures of patent document 1 are provided to carry out an eyesight examination by selecting a specific direction recognized by such optotypes, but a subject with astigmatism or the like has difficulty in specifying a correct direction due to astigmatism, thereby failing to perform accurate eyesight examination.

In addition, the eyesight examination is typically performed by using various types of optotypes (for example, numbers, letters, Landolt rings, ships, airplanes, umbrellas and animal patterns.), and thus it is difficult to carry out accurate examination by using only Landolt optotypes or the figures of patent document 1.

In order to carry out the eyesight examination by using various types of optotypes, it is necessary to have an accurate optotype identification by a subject. However, there is still a need to configure an eyesight examination so that an accurate eyesight examination is performed by a simple identification.

It would also be desirable to configure an eyesight examination so that the identification by a subject can be intuitively inputted without the need for further explanation.

As mentioned above, an eyesight examination method, an eyesight examination device and a downloader server for storing a program of the eyesight examination method are need to solve the various problems caused by the known eyesight examination methods.

### [Disclosure]

### [Technical Problem]

The present invention has been derived to solve the problems mentioned as above and has an objective to provide an eyesight examination method, an eyesight examination device and a downloader server for storing a program of the eyesight examination method, in which automatic eyesight examination can be carried out using various types of optotypes.

The present invention has another objective to provide an eyesight examination method, an eyesight examination device and a downloader server for storing a program of the eyesight examination method, in which an accurate eyesight examination can be carried out by allowing a user to select a response to the various types of displayed optotypes, irrespective of eye refraction such as astigmatism and the like.

In addition, the present invention has still another objective to provide an eyesight examination method, an eyesight examination device and a downloader server for storing a program of the eyesight examination method, in which a user is allowed to intuitively select a response to an image displayed for eyesight examination, regardless of age, such that automated eyesight examination can be performed without any particular expression of the user's intention.

The technical problems to be solved by the present invention are not limited to the technical problems mentioned above, and other technical problems not mentioned above may be clearly understood by a person skilled in the art, to which the present invention belongs, from the following description.

### [Technical Solution]

According to one aspect of the present invention, an eyesight examination method according to claim 1 is provided.

As for the eyesight examination method, each of the displayed plurality of reference images is positioned outside the displayed optotype image in different directions from each other, the response includes a direction input for selecting one reference image from among the plurality of reference images, and step (c) is to determine whether the reference image corresponding to the direction input of the response and the optotype image are identical with each other.

As for the eyesight examination method, the size of the reference images is equal to or greater than the size of the largest one of optotype images having the identical shape.

The eyesight examination method described above further includes step (d) of displaying a subsequent optotype image selected according to the determination result of step (c) and the plurality of reference images, wherein in steps (a) and (d), for binocular examination of a subject, two optotype images are displayed and two sets of the plurality of reference images are displayed to be positioned outside each of the optotype images, respectively.

According to another aspect of the present invention, an eyesight examination device according to claim 4 is provided.

As for the eyesight examination device, the plurality of reference images displayed on the display unit are respectively positioned outside the displayed optotype image in different directions from each other, the response received through the input unit includes a direction input for selecting one reference image from among the plurality of reference images, and the control unit determines whether the reference image corresponding to the direction input of the response among the plurality of reference images and the optotype image are identical with each other.

As for the eyesight examination device, the control unit selects a subsequent optotype image according to the determination result of the determined reference image and the optotype image and outputs the selected subsequent optotype image and the plurality of reference images through the display unit, and, for binocular examination of a subject, the display unit displays two optotype images and two sets of the plurality of reference images positioned outside each of the optotype images respectively.

As for the eyesight examination device, the display unit includes two display modules for displaying the optotype images respectively and a reference optotype plate for displaying each of the plurality of reference images and accommodating the two display modules.

As for the eyesight examination device, the display unit includes two display modules for displaying the optotype images respectively and corresponding plurality of reference images, and the control unit carries out control such that each of the plurality of reference images has a size equal to or greater than the size of the largest one of the optotype images having an identical shape and is outputted together with the optotype image to the display module.

According to a further aspect of the present invention, a downloader server for storing programs of an eyesight examination method according to claim 7 is provided.

### [Advantageous Effects]

The eyesight examination method, the eyesight examination device and the downloader server which stores the programs of the eyesight examination method according to the present invention as described above have the effect of enabling automatic eyesight examination by using various types of optotypes.

In addition, the eyesight examination method, the eyesight examination device and the downloader server which stores the programs of the eyesight examination method according to the present invention as described above have the effect of allowing a user to select a response to the various types of displayed optotypes, irrespective of eye refraction such as astigmatism and the like, so that accurate eyesight examination can be performed.

In addition, the eyesight examination method, the eyesight examination device and the downloader server which stores the programs of the eyesight examination method according to the present invention as described above have the effect of allowing a user to intuitively select a response to an image displayed for eyesight examination, regardless of age, thereby enabling automated eyesight examination to be performed without any particular expression of the user's intention.

The effects obtained by the present invention are not limited to the effects mentioned above, and other effects not mentioned above may be clearly understood by a person skilled in the art, to which the present invention belongs, from the following description.

### [Description of Drawings]

Fig. 1 is an exemplary block diagram of an eyesight examination device.
Fig. 2 is a view showing an exemplary appearance of an eyesight examination device.
Fig. 3 is a view showing a display unit including a single display module of a graphic pixel type.
Fig. 4 is a view showing a display unit including two display modules of a graphic pixel type.
Fig. 5 is a view showing a display unit including a display module of a segment type, and
Fig. 6 is a view showing an exemplary flow chart of an eyesight examination.

### <Brief Explanation of Reference Symbols>

| | | | |
|---|---|---|---|
| 100: | eyesight examination device | | |
| 101: | input unit | 103: | storage unit |
| 105: | external interface unit | 107: | output unit |
| 109: | sensing unit | 111: | display unit |
| 111-1: | display module | | |
| 111-3: | reference optotype plate | | |
| 113: | illumination unit | 115: | optical unit |
| 117: | control unit | 119: | connection unit |

### [Best Mode for Carrying out the Disclosure]

Hereinafter, an embodiment of the present invention will be described in more detail with reference to the accompanying drawings.

In the drawings, like reference numerals refer to like elements.

The above and other objects, features and advantages of the present invention will become more apparent from the following detailed description of the present invention when taken in conjunction with the accompanying drawings, and those skilled in the art to which the present invention belongs will be able to easily implement the technical idea of the present invention. Also, in describing the present invention, a detailed description of known technology related to the present invention will be omitted if it is judged that the gist of the present invention may be unnecessarily blurred. Hereinafter, preferred embodiments according to the present invention will be described in detail with reference to the accompanying drawings.

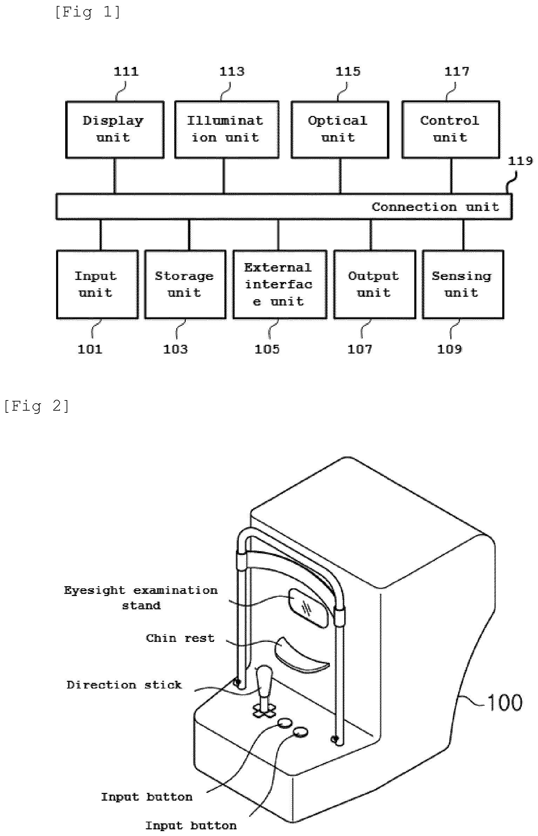

Fig. 1 is an exemplary block diagram of an eyesight examination device 100.

Referring to Fig. 1, the eyesight examination device 100 includes an input unit 101, a storage unit 103, an external interface unit 105, an output unit 107, a sensing unit 109, a display unit 111, an illumination unit 113, an optical unit 115, a control unit 117 and a connection unit 119.

The blocks shown in Fig. 1 may be omitted depending on the types of the eyesight examination device 100 or design changes thereof, and other blocks not shown in Fig. 1 may be further included in the block diagram.

The eyesight examination device 100 may be of various types. For example, the eyesight examination device 100 may be a mobile phone, a smart phone, a tablet PC, a personal computer and the like, or a server capable of performing an eyesight examination through the connection to a network such as the Internet, or a dedicated terminal configured for an eyesight examination (see Fig. 2) .

Depending on the type of the eyesight examination device 100, a predetermined block may be omitted, wherein if the eyesight examination device 100 is, for example, a personal computer, a smart phone, a tablet PC or the like, it is possible to omit the illumination unit 113, the optical unit 115 and/or the sensing unit 109 and the like depending on the configuration of each device.

Referring to Fig. 1 with respect to each block of the eyesight examination device 100 on the assumption that the eyesight examination device 100 is a dedicated terminal, the input unit 101 receives a user input from a subject. The input unit 101 is provided with a direction stick for selecting 2-direction, 4-direction or 8-direction, one or more buttons, a touch panel, a touch pad, a mouse, a keyboard, and the like so as to receive a user input and the user input is outputted to the control unit 117.

The user input received through the input unit 101 can represent a response to the optotype image displayed for an eyesight examination. The response to the optotype image may include a direction input indicating, for example, a direction from the optotype image to a selected reference image, so as to select one of the plurality of reference images displayed together with the optotype image. This response can be either a direction input itself or a signal for extracting a direction input.

In addition, the input unit 101 may be used for controlling the eyesight examination device 100 by a user. For example, the input unit 101 may receive a user input requesting the operation of the eyesight examination device 100, the start and end of an eyesight examination program of the eyesight examination device 100, the output of a result of the eyesight examination, and the like, and may transmit the input to the control unit 117.

The storage unit 103 stores various data and programs. The storage unit 103 includes a volatile memory, a non-volatile memory and/or a mass storage medium such as a hard disk, and the like. For example, the storage unit 103 stores the eyesight examination programs used for eyesight examinations. The eyesight examination program can be downloaded from a downloader server over a network. The eyesight examination program is loaded into the control unit 117 to perform the eyesight examination of a user(subject) and to generate and output eyesight examination results.

Further, the storage unit 103 can store various data used for eyesight examinations, for example, a plurality of optotype image groups used for eyesight examinations. Each of the optotype image groups includes different optotype images for identical shapes. For example, one optotype image group includes Landolt optotype images of different sizes, and the other one group of the optotype images includes optotype images that can be used for eyesight examinations and which represent specific number, specific letter or specific picture(for example, umbrellas, boats, airplanes, and so on) having identical shapes. Another group of the optotype images include optotype images representing number, letter, or picture, etc. having shapes different from those of the previous group of the optotype images. Thus, the optotype image groups include the optotype images of identical shapes and different sizes with each other.

The optotype image groups include a plurality of optotype images of different sizes and reference image of the identical shape with those of the optotype images. Each of the optotype images corresponds to a specific eyesight data (an eyesight value, for example, 0.1, 0.2, 2.0, etc.) and the reference image has identical shapes with those of the optotype images and are equal to or greater than the largest one of the optotype images. The reference image can be displayed and used for user response.

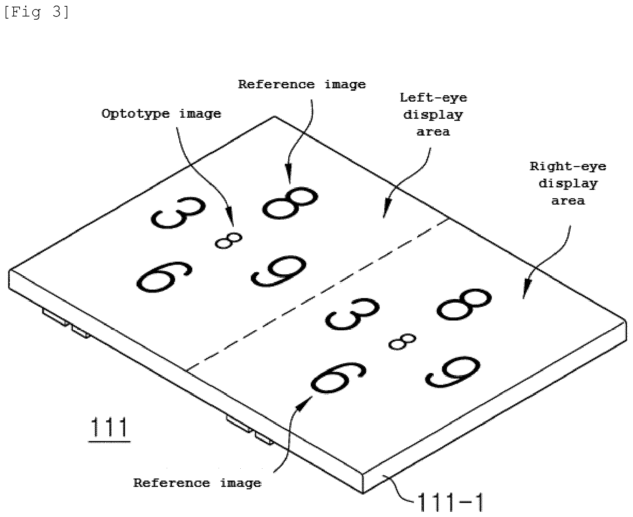

As shown in Fig. 3 to Fig. 5, in which various features of the display unit 111 are illustrated, the groups of the optotype images may be pre-stamped (imprinted or drawn) on the display unit 111 instead of being stored in the storage unit 103, and one or more optotype images of a predetermined optotype image group in the one or more optotype image groups and the reference images of the predetermined optotype image group can be displayed according to a control signal by the control unit 117.

The external interface unit 105 is an interface coupling with a device outside the eyesight examination device 100. The external interface unit 105 can perform short-range communication so as to interface with a remote controller, facsimile equipment, a personal computer and the like. The external interface unit 105 includes an interface for an infrared remote control signal, or a communication interface for Ethernet, Bluetooth, or FAX, and thus can transmit or output an eyesight examination result under the control of the control unit 117. In addition, the external interface unit 105 can receive control data from an external device and transmit the same to the control unit 117. The control unit 117 can perform operations according to the received control data.

The output unit 107 outputs an eyesight examination result generated by the control unit 117. The output unit 107 includes a display module such as an LCD, an LED or the like, a printer module that can be embedded in the casing of the eyesight examination device 100, and/or an interface for interfacing with an external printer.

The output unit 107 may further include a speaker. The speaker can output a voice to inform of an eyesight examination result and can output the announcement to guide the start of an eyesight examination or operations, action or the like to be taken by a subject(user) under the control of the control unit 117.

The sensing unit 109 recognizes a subject or a user of an eyesight examination. Depending on the subject recognition by the sensing unit 109, an eyesight examination may be initiated by the control unit 117. The sensing unit 109 may include a pressure sensor for sensing the pressure applied to a chin rest, a temperature sensor for sensing the body temperature of a subject or a user sensed by an eyesight examination stand, an infrared sensor capable of recognizing a subject or a user, a proximity sensor for recognizing the proximity of a subject or a user and/or a circuit for outputting a signal that changes according to the pressure applied to the chin rest, and the like.

Herein, both the terms "a subject" and "a user" can be used throughout the description of the present invention. Strictly speaking, a person who is undergoing an eyesight examination according to the induction or guidance of another person is recognized as "a subject", and a person who performs an eyesight examination by himself by using the eyesight examination device 100 can be recognized as "a user". Hereinafter, both types of the users are referred to as "a subject".

The signal sensed by the sensing unit 109 is outputted to the control unit 117 and the control unit 117 can start an eyesight examination according to the type of the eyesight examination device 100, load an eyesight examination program, or provide other guidance to a subject.

The display unit 111 displays an image. The display unit 111 includes one or more display modules 111-1 and preferably includes two display modules 111-1.

When the display unit 111 is composed of two display modules 111-1, one display module 111-1 (hereinafter, referred to as "a left-eye display module") displays an image for checking the eyesight of the left eye (hereinafter, referred to as "left-eye") and the other one display module 111-1 (also referred to as "a right-eye display module") displays an image for checking the eyesight of the right eye (referred to as "right-eye").

the corresponding left-eye display module 111-1 is used to check the left-eye eyesight, the corresponding right-eye display module 111-1 is used to check the right-eye eyesight, and the both display modules 111-1 are used to check the binocular eyesight of the both eyes. The relative positions of the left-eye display module 111-1 and the right-eye display module 111-1 in the eyesight examination device 100 may be changed according to the structure and the like of the lens and/or the reflection mirror of the optical unit 115.

The display unit 111 may be composed of a single display module 111-1. In this case, the single display module 111-1 may be used for the eyesight examination of the left eye, the right eye and/or the both eyes.

The display module 111-1 may be an LCD module or an LED module. Each display module 111-1 receives an image or video signal from the control unit 117 through a predetermined interface and displays the image (the video) according to the received signal.

The display module 111-1 may be an LCD module of a graphic pixel type. In the case of such a graphic pixel type LCD module, the pixel size of this LCD module is preferably 0.04 mm or less. In order to cover the result range (for example, 0.1 to 2.0) of the eyesight examination and to carry out an eyesight examination, the graphic pixel size should be 0.04 mm or less.

The display module 111-1 may be an LCD module of a segment type. The segment type LCD module can display precise optotypes (images) used for eyesight examinations by pre-drawing (imprinting) specific patterns and shapes, which are optotype images used for eyesight examinations. Such a segment type LCD module is configured to pre-draw the optotype images of one or plurality of groups of optotype images and to display a specific optotype image under the control of the control unit 117. For example, the segment-type LCD module can highlight a specific optotype image according to a control signal from the control unit 117 so as to display the specific optotype image and display (highlight) another specific optotype image according to another control signal.

The display unit 111 will now be described in more detail. Fig. 3 to Fig. 5 are views showing the configuration of various types of display units 111.

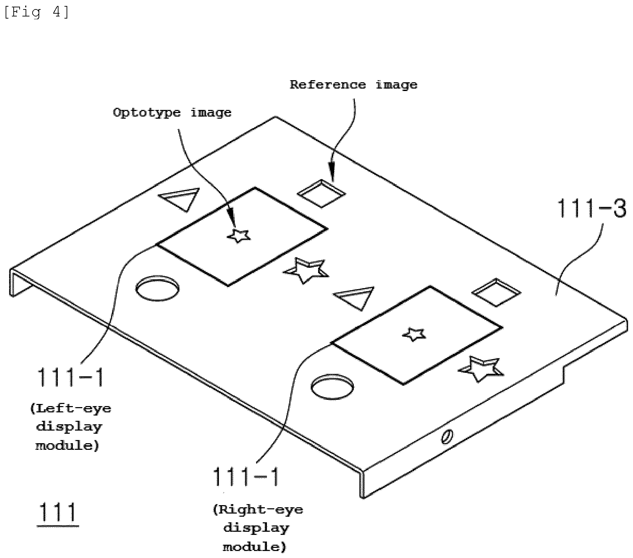

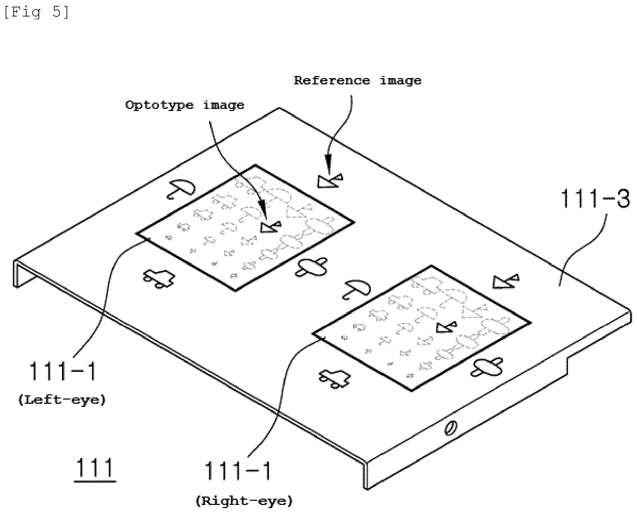

Fig. 3 is a view showing the display unit 111 including the single display module 111-1 of the graphic pixel type, Fig. 4 is a view showing the display unit 111 including the two display modules 111-1 of the graphic pixel type, and Fig. 5 is a view showing a display unit 111 including a segment type display module 111-1.

As shown in Fig. 3 to Fig. 5, the display unit 111 displays an optotype image used for a current eyesight examination and a plurality of reference images used to generate a subject's response to this optotype image.

An optotype image is selected by the control unit 117 from the optotype image groups (of the storage unit 103 or pre-drawn) and the reference images are located outside the optotype image. For example, one of two reference images may be located on the left side of the optotype image and the other reference image may be located on the opposite side. For example, one of four reference images may be located on the left side of the optotype image, another reference image may be located on the right side of the optotype image, still another reference image may be located on the lower side of the optotype image, and the other reference image may be located on the upper side of the optotype image.

One of the displayed reference images is the identical image with the currently displayed optotype image. That is, only one reference image of the reference images is identical with the displayed optotype image, and the other reference images are different from the currently displayed optotype image.

As can be seen in Fig. 3, the display unit 111 may be composed of the single display module 111-1 and this single display module 111-1 may be the graphic pixel LCD module or the graphic pixel LED module. In this case, the display module 111-1 can be divided into two areas. One display area is the display area for the left-eye eyesight examination and the other display area can be the display area for the right-eye eyesight examination. These two display areas are used simultaneously for binocular eyesight examination (see Fig. 3).

At the time of the binocular eyesight examination, the optotype image and the plurality of the 2-direction or 4-direction reference images displayed on the left-eye display area are the same as the optotype image and the plurality of the reference images displayed in the right-eye display area each other.

As can be seen in Fig. 4 showing another example, the display unit 111 may be composed of the two display modules 111-1 and each of the display modules 111-1 may be the graphic pixel LCD module or the graphic pixel LED module.

One display module 111-1 is used as the left-eye display module 111-1 for displaying an optotype image at the time of the left-eye eyesight examination, and the other display module 111-1 is used as the right-eye display module 111-1 for displaying an optotype image at the time of the right-eye eyesight examination. Both the display modules 111-1 display the same optotype image so that the display unit 111 is configured to examine binocular eyesight.

The display unit 111 of Fig. 4 may further include a reference optotype plate 111-3. The reference optotype plate 111-3 can accommodate the two display modules 111-1. That is, the reference optotype plate 111-3 can be coupled through the holes provided in the reference optotype plate 111-3 and further can be detached therefrom. It is also possible to couple a different reference optotype plate 111-3 may be additionally coupled to the display unit 111 or the eyesight examination device 100. The reference optotype plate 111-3 includes two holes (square holes) capable of accommodating the two display modules 111-1 and can be attached to or detached from the two display modules 111-1 through the two holes. The reference optotype plate 111-3 can be fixed to and separated from the inside of the eyesight examination device 100 by means of a coupling means (for example, a screw, an adhesive, a mechanical structure for coupling or the like).

The reference optotype plate 111-3 can display a plurality of reference images. As can be seen from Fig. 4, the reference optotype plate 111-3 can display reference images in the top, bottom, left, and right directions of each display module 111-1. The reference optotype plate 111-3 may display the reference images, which are printed or imprinted by embossing or engraving the same. In this case, during the eyesight examination, corresponding reference images can be recognized by a subject through LED illumination or the like.

It is possible to omit the reference optotype plate 111-3 and display all the reference images to be located outside the optotype image by means of each display module 111-1 according to the size or design variation of the display module 111-1.

As can be seen in Fig. 5 showing another example, the display unit 111 includes the two display modules 111-1 and the reference optotype plate 111-3. Each of the display modules 111-1 is the segment type LCD module.

One display module 111-1 is used as the left-eye display module 111-1 for displaying an optotype image at the time of the left-eye eyesight examination, and the other display module 111-1 is used as the right-eye display module 111-1 for displaying an optotype image at the time of the right-eye eyesight examination. Both the display modules 111-1 display the optotype image so that the display unit 111 is configured to examine binocular eyesight.

The segment LCD module can include all the pre-drawn (imprinted) optotype images of the one or more optotype image groups and display one specific optotype image according to the control signal selected by the control unit 117. For example, the control unit 117 outputs a control signal to power the selected optotype image so that this segment LCD module can display the selected specific optotype image.

The display unit 111 of Fig. 5 may further include the reference optotype plate 111-3, wherein the reference optotype plate 111-3 is configured to accommodate two display modules 111-1. Each display module 111-1 can be attached to or detached from the reference optotype plate 111-3 through a provided hole (for example, a square hole) or the like. Another reference optotype plate 111-3, representing other reference images, may be coupled to the display unit 111 or the eyesight examination device 100. The reference optotype plate 111-3 can be fixed to and separated from the inside of the eyesight examination device 100 by means of a coupling means (for example, a screw, an adhesive, a mechanical structure for coupling or the like).

The reference optotype plate 111-3 can display a plurality of reference images. As can be seen from Fig. 4 or Fig. 5, the reference optotype plate 111-3 can display reference images in the top, bottom, left, and right directions of each display module 111-1. The reference optotype plate 111-3 may include printed reference images or display the reference images through embossing, engraving or the like. In this case, during the eyesight examination, corresponding reference images of the reference optotype plate 111-3 can be recognized by a subject through LED illumination or the like.

The two segment LCD modules of Fig. 5 can be attached to or detached from the eyesight examination device 100. For example, the two segment LCD modules can be replaced with new segment LCD modules, which can display the optotype images of other optotype image groups, in accordance with circumstances such that the new segment LCD modules can be installed in the eyesight examination device 100.

As can be seen in Fig. 3 to Fig. 5 showing the above various configuration examples, the display unit 111 displays the optotype images used for the eyesight examination under the control of the control unit 117 and further displays the plurality of reference images, wherein only one reference image among the plurality of reference images has the identical shape with that of the currently displayed optotype image. For example, if the optotype image has the shape of the number 8, an umbrella, a ship, or an airplane, one of the plurality of reference images has the shape of the number 8, an umbrella, a ship, or an airplane, which is the same as the optotype image.

The size of the reference images is equal to or greater than the size of the largest optotype image among the same shaped optotype images. Alternatively, it is recognized by a subject that the displayed reference image is equal to or greater than the largest optotype image.

Referring back to the blocks of Fig. 1, the illumination unit 113 outputs light to the display unit 111. The illumination unit 113 includes a plurality of white or colored LEDs to allow the optotype image and, by extension, the reference images displayed in the display modules 111-1 of the display unit 111 to be maintained at a constant brightness.

Considering the configuration of the illustrative illumination unit 113 corresponding to the display unit 111 in more detail, if the display unit 111 includes the reference optotype plate 111-3, the illumination unit 113 includes a plurality of LEDs for illuminating the reference images of the reference optotype plate 111-3. One or more LEDs of the plurality of LEDs for illuminating the reference optotype plate 111-3 illuminate the left-eye side reference images of the reference optotype plate 111-3 and are controlled by the control unit 117. Further one or more LEDs of the plurality of LEDs for illuminating the reference optotype plate 111-3 illuminate the right-eye reference images of the reference optotype plate 111-3 and are controlled according to a control signal by the control unit 117. In addition, the illumination unit 113 also includes one or more LEDs for illuminating each display module 111-1. If the display module 111-1 itself includes LEDs, the LEDs for illuminating the display module 111-1 may be omitted.

The optical unit 115 includes one or more lenses and reflection mirrors so that the subject's eyes can recognize the optotype image and the plurality of reference images of the display unit 111 in the eyesight examination device 100. Depending on the equipment type of the eyesight examination device 100, the optical unit 115 may be omitted.

The control unit 117 controls each block of the eyesight examination device 100. The control unit 117 uses the eyesight examination programs and the data (for example, the optotype images and the like) stored in the storage unit 103 and processes the data according to the programs so as to control other blocks.

The control unit 117 responds to the input of a subject or the input of a manager of the eyesight examination device 100 through the input unit 101, processes the data according to the input, and outputs the processing result through the output unit 107. The control unit 117 includes one or more execution units capable of executing program codes and may represent or be referred to as a processor, a microcomputer, a CPU, an MPU, or the like.

The control unit 117 loads an eyesight examination program according to the input received through the input unit 101 and sets an eyesight examination mode for the both eyes, the left eye and/or the right eye of a subject in the eyesight examination program, and can perform an eyesight examination in the designated examination mode by the subject recognition through the sensing unit 109.

In the left-eye eyesight examination, the control unit 117 controls the left-eye display module 111-1 and, by extension, the reference optotype plate 111-3 and thus displays the optotype image and the plurality of reference images. In the right-eye eyesight examination, the control unit 117 controls the right-eye display module 111-1 and, by extension, the reference optotype plate 111-3 and thus displays the optotype image and the plurality of reference images. In the eyesight examination of the both eyes, the control unit 117 controls the right-eye and left-eye display modules 111-1 and, by extension, the reference optotype plate 111-3 and thus displays each of the plurality of reference images.

After the display of the optotype image in the eyesight examination procedure, the control unit 117 can receive the response of the subject via the input unit 101. The subject inputs the response to the optotype image through the input unit 101 according to the recognition of the displayed optotype image and the plurality of reference images, and the input unit 101 receives the input signal representing the response.

For example, the subject recognizes a reference image that has the identical shape with that of the optotype image and can input a response indicating the direction from the optotype image to the same reference image by means of a direction stick, a button, or the like. The input unit 101 receives a signal indicating this response from the direction stick, the button, or the like.

Upon receipt of the response, the control unit 117 determines a reference image selected from the response among the displayed plurality of reference images and determines whether the determined reference image and the currently displayed optotype image are the same shape.

For example, the control unit 117 receives a direction input through the input unit 101 and determines a reference image corresponding to the direction input among a plurality of 2-direction, 4-direction, or 8-direction reference images, and determines whether the corresponding reference image is the same as the current optotype image. The control unit 117 can determine the direction of the reference image having the identical shape with that of the currently displayed optotype image and can make this determination if the direction input and a preset direction are the same.

The control unit 117 determines a subsequent optotype image according to the determination result of the sameness. For example, if it is determined that they are the same, the control unit 117 may select an optotype image having an eyesight value smaller than an eyesight value corresponding to the currently displayed optotype image. Otherwise, control unit 117 may select an optotype image having an eyesight value greater than an eyesight value corresponding to the currently displayed optotype image. The selected optotype image and the currently displayed optotype image may have the same shape or different shapes.

In addition, the control unit 117 can determine a plurality of reference images to be used for this subsequent optotype image according to the selection of the subsequent optotype image. The determination of the reference images may be omitted according to the configuration example of the display unit 111 (for example, the reference optotype plate 111-3).

According to the determination of the subsequent optotype image and the plurality of reference images, the control unit 117 outputs an image (video) signal representing the subsequent optotype image and, by extension, the plurality of reference images to the display unit 111.

In a monocular examination (for example, the left-eye or right-eye eyesight examination), the control unit 117 outputs a single optotype image and a plurality of reference images displayed outside the optotype image through the display unit 111. In a binocular test, the control unit 117 outputs two optotype images and a plurality of reference images displayed outside of each of the optotype images through the display unit 111.

The connection unit 119 enables the transmission and reception of the data/control data between the respective blocks. The connection unit 119 is composed of one or more combinations of a parallel bus, a serial bus, general purpose input/output GPIO, or the like.

Fig. 2 is a view showing an exemplary appearance of the eyesight examination device 100.

The eyesight examination device 100 of Fig. 2 is an example of a dedicated terminal configured according to the present invention. Referring to Fig. 2, the eyesight examination device 100 includes the chin rest, the eyesight examination stand, and the direction stick and one or more buttons included in the input unit 101. The direction stick or one particular button may be omitted in accordance with a modification example or replaced by other input means for performing the same or similar function.

Briefly considering the appearance of the eyesight examination device 100, the chin rest is an instrument for seating the chin of a face during the examination, the eyesight examination stand is configured to accommodate both eyes and an image is displayed to be exposed through one eye or both eyes through the eyesight examination stand.

The direction stick is configured to point to a specific direction, in which the same reference image as the optotype image displayed through the eyesight examination stand is represented. For example, the direction stick outputs a direction signal indicating the left, right, up or down direction to the control unit 117 of the eyesight examination device 100.

The button is also used to enter a subject's response to the displayed optotype image. For example, the button can be used to indicate that a subject does not recognize the optotype image or cannot find the same reference image.

It is possible to use the button only, or the direction stick only or the both button and direction stick for an eyesight examination. Depending on the use of the eyesight examination, the button or the direction stick may be omitted. It is possible to employ a push-type button or a touch-type button as the button.

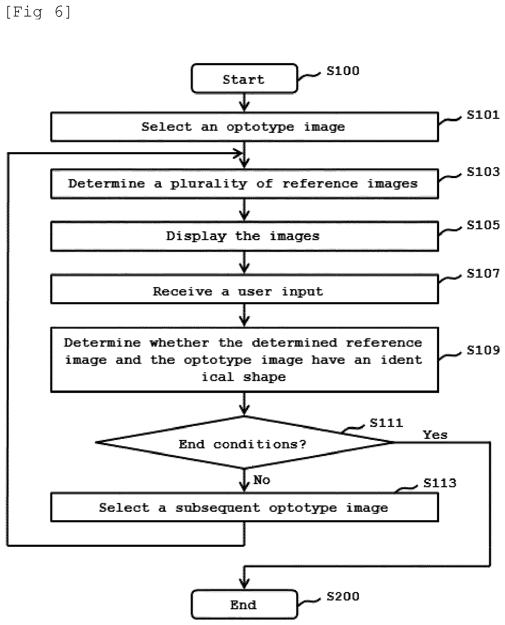

Fig. 6 is a view showing an exemplary flow chart of an eyesight examination.

The routine of Fig. 6 is performed by the eyesight examination device 100 and preferably by controlling the other blocks by using the eyesight examination program loaded by the control unit 117. The eyesight examination program can be used for various purposes but is a program that can examine the eyesight of a subject(user) by using at least the input unit 101, etc. The eyesight examination program can be configured to perform an eyesight examination, for example, in the form of a game while providing a child with a moving picture and voice. Alternatively, the eyesight examination program is embedded in a game program for a child or a teenager so as to enable the child or the teenager to be provided with an eyesight examination function while playing the corresponding game. The eyesight examination program can be a dedicated program for eyesight examinations or a program that can be embedded in any other types of programs so as to perform eyesight examinations at a certain point in time.

Since eyesight examination device 100 has already been described in detail with reference to Figs. 1 to 5, the overlapping features will be briefly described.

First, in a set left-eye, right-eye or binocular eyesight examination mode, the control unit 117 selects one optotype image from the plurality of optotype image groups (S101). For example, the control unit 117 selects one optotype image group from among the plurality of optotype image groups of numbers, letters, umbrella shapes, ship shapes, airplane shapes, and the like, and selects an optotype image corresponding to a specific eyesight value in the selected optotype image group. For example, the control unit 117 may select an optotype image corresponding to an intermediate eyesight value within an eyesight range, for which an eyesight examination can be carried out, in the optotype image group.

In addition, the control unit 117 determines a plurality of reference images (S103). Step S103 may be omitted or preceded by step S101 according to the configuration examples of the display unit 111. For example, if the reference optotype plate 111-3 to be attached to or detached from the display module 111-1 is used, the reference optotype plate 111-3 to be used first by a manager of the eyesight examination device 100 can be coupled to the display module 111-1. At least the shape of one reference image of the plurality of reference images is the same as the shape of the selected optotype image. The reference image having the same shape has a size equal to or greater than the size of the largest optotype image in the optotype images of the selected optotype image group.

The reference images have the largest size, which is larger than the size of the optotype images so that a subject can accurately recognize the shapes of the reference images. For example, the reference images are formed to be equal to or greater than the size of the optotype image corresponding to 0.1 that is the lowest possible eyesight value for the eyesight examination.

Thereafter, the control unit 117 controls the display unit 111 such that the display unit 111 displays the selected optotype image and the plurality of reference images to be recognized by the subject (S105). According to the left-eye, right-eye, and binocular eyesight examination modes, the control unit 117 displays the optotype image and the plurality of reference images in the left-eye display area and displays the optotype image and the plurality of reference images in the right-eye display area. In the binocular eyesight examination mode, the control unit 117 displays two identical optotype images and two identical sets of reference images in the left-eye display area(module) and the right-eye display area(module). The plurality of reference images can be displayed outside the optotype images and a subject can determine the direction of each reference image from the optotype image.

The control of the control unit 117 for displaying the optotype image and the plurality of reference images may be different depending on the configuration of the display unit 111.

When the display unit 111 is composed of the single display module 111-1 of the graphic pixel type, the control unit 117 forms an image frame by using the pixel data of the optotype image and the pixel data of the determined plurality of reference images from the storage unit 103, and outputs the video signal of thus formed image frame to the single display module 111-1. In the binocular eyesight examination, the two identical optotype images and two identical sets of the plurality of reference images are displayed in the image frame. In the monocular left-eye or right-eye eyesight examination, a single optotype image and a plurality of reference images positioned outside the optotype image are displayed in the corresponding display area in the image frame.

When the display unit 111 is composed of the two display modules 111-1 of the graphic pixel type, an image frame for the corresponding display module 111-1 is formed according to a monocular or binocular eyesight examination mode and video signal of the formed image frame is outputted to the corresponding display modules 111-1. The control unit 117 may form one or two image frames, and each image frame includes an optotype image and, by extension, may further include a plurality of reference images. When the reference optotype plate 111-3 is coupled to the display unit 111, a plurality of reference images are not included in the image frame and the control unit 117 controls the illumination unit 113 so as to display the plurality of reference images of the reference optotype plate 111-3 such that one or more LEDs corresponding to the plurality of reference images are turned on.

When the display unit 111 is composed of the display module 111-1 of the segment type, the control unit 117 outputs a control signal to one or two display modules 111-1 so as to display a selected optotype image (for example, to provide highlight or illumination of the corresponding optotype image). In addition, in order to display each of the plurality of reference images for the reference optotype plate 111-3, the control unit 117 controls the illumination unit 113 such that one or more LEDs corresponding to one or two sets of the plurality of reference images selected from the two sets of the plurality of reference images according to the eyesight examination mode. The LEDs can output light to the corresponding reference images and thus a subject can recognize thus displayed reference images.

Regardless of the type of display unit 111, the displayed reference images are all located outside the displayed optotype image. That is, the reference images are located outside the displayed optotype image in a variety of directions, which can be selected in association with the direction stick or the input button (for example, up, down, right and left selection buttons) of the input unit 101. The respective reference images are located outside the optotype image in different directions from each other.

In this manner, the control unit 117 can display the optotype image and the plurality of reference images, and the subject can recognize these images. Accordingly, the user inputs a response to the displayed optotype image through the input unit 101, and the control unit 117 receives the user input indicating the response to the optotype image through the input unit 101 (S107).

Thereafter, the control unit 117 determines a reference image selected from the displayed plurality of reference images by receiving the user input representing the response to the optotype image, and determines whether the determined reference image and the currently displayed optotype image have an identical shape or not (S109). The response is the user input that indicates the selection for the reference image that has the identical shape with that of the optotype image. To induce accurate subject selection, only one of the plurality of reference images is identical with the currently displayed optotype image.

Speaking in detail with reference to an example, the subject recognizes the optotype image through his eyes and also recognizes the plurality of images located outside the optotype image. The subject can recognize the reference image having the identical shape with that of the optotype image by comparing each of the plurality of images with the optotype image. In order to represent the recognized reference image, the subject can provide the user input through the input unit 101 so as to select the reference image recognized in the identical shape. For example, the subject can input, through the input unit 101, the response, which includes the direction input indicating the corresponding direction, by using the direction stick and the like in order to select the identical reference image located outside in two directions, four directions, or eight directions around the optotype image.

Upon receipt of the direction input of the response, the control unit 117 determines the reference image corresponding to the direction input from among the plurality of reference images and determines whether the corresponding reference image is identical with the currently displayed optotype image. For example, the control unit 117 can determine whether the reference image of the direction input, which is selected by the subject, is identical with the displayed optotype image by comparing the direction value of the received direction input with the direction value of the reference image having the identical shape in a state, in which the direction value of the reference image having the identical shape is determined in advance (in step S103 or step S105).

Then, the control unit 117 determines end conditions of the eyesight examination (Sill). The end conditions may be the number of repetitions of the optotype image display (S105), whether an accurate eyesight examination result is obtained according to user inputs, and the like. When a correct eyesight result is obtained, the control unit 117 can output the eyesight examination result through the output unit 107 or the external interface unit 105.

If the end condition is met, the routine of Fig. 6 is ended in step S200 by the control unit 117.

If the end condition is not met, the control unit 117 selects an optotype image to be displayed subsequently according to the previous response reception and determination (S113). If it is determined to be an identical shape in the previous determination (S109), the control unit 117 selects a smaller optotype image corresponding to an eyesight value (for example, 1.2) higher than the eyesight value (for example, 1.0) corresponding to the previously displayed optotype image. If it is not determined to be an identical shape, the control unit 117 selects a larger optotype image corresponding to an eyesight value (for example, 0.8) lower than the eyesight value (for example, 1.0) corresponding to the previously displayed optotype image.

According to the selection of the subsequent optotype image, the control unit 117 may repeat step S103 to step S109, thereby performing the correct eyesight examination of the subject. Through step S105, the control unit 117 can display the subsequent optotype image and a plurality of reference images to be used in the selection of the subsequent optotype image.

As described above, the eyesight examination method according to the present invention can perform the eyesight examination with the user input indicating whether the shapes of the reference images and the optotype image, which are recognized by the subject, are identical. Furthermore, the eyesight examination is performed using the direction input for the selection of the reference image around the optotype image, and thus anyone can easily perform the eyesight examination. In addition, the eyesight examination method according to the present invention is similar to a shape matching game and thus can add fun to the eyesight examination, and can perform the eyesight examination during the reproduction of any other game or contents in connection therewith.

According to the present invention, it is possible to perform an accurate eyesight examination by using the images of various shapes regardless of whether the subject has an issue of astigmatism or the like and, by extension, it is possible to perform an astigmatism test or the like. According to the present invention, since the subject can simply and intuitively provide an input for the eyesight examination through the image comparison and the uniform input method to indicate an identical image, the eyesight examination can be performed for subjects in various categories.

Meanwhile, the eyesight examination program for executing the routine of Fig. 6 can be downloaded from a downloader server through a network.

The downloader server includes at least a communication module, a storage module and a control module for controlling the downloading of a requested program.

The communication module receives a download request through a network and transmits a program (for example, an eyesight examination program) corresponding to the download request through the network to a requested device (for example, the eyesight examination device 100). The communication module can handle such data transmitting and receiving by including a chipsets for processing one or more wired or wireless LANs.

The storage module has a mass storage medium such as a hard disk and stores a plurality of programs that can be downloaded. The storage module stores at least the eyesight examination program for performing the eyesight examinations according to the present invention (see Fig. 6).

The control module includes one or more execution units so as to search for a program stored in the storage module according to a download request received through the communication module, extract the requested program, and transmit the extracted program as a response to the request to a requested device through the communication module.

For example, the control module can extract the eyesight examination program from the storage module according to the download request for the eyesight examination program, transfer the extracted eyesight examination program to the communication module, and transmit the same to the requested eyesight examination device 100.

The optotype images displayed in the present invention can be various types of images. The optotype images can be numbers, figures, letters, or the like. Alternatively, the optotype images may be color vision(sense) test images used in the examination of color weakness or color blindness.

The eyesight examination method and the eyesight examination device 100, according to the present invention, can perform the color vision(sense) test alternatively or together with any other eyesight examination by confirming a response through a user input. In order to examine the recognition of specific colors, the optotype images used in the color vision(sense) test are composed of dots of various colors and specific numbers are displayed in specific colors. If the optotype image is a color vision(sense) test image, one of a plurality of displayed reference images represents a number identical with the number displayed by the dots of the same color in the optotype image. The reference images can be displayed, for example, in black.

During the color vision(sense) test, the eyesight examination method and the eyesight examination device 100 can use two display modules 111-1 or a single display module 111-1 and perform the color vision(sense) test for one eye or both eyes.

## Claims

1. An eyesight examination method to be carried out in an eyesight examination device (100) according to claim 4, comprising the steps of:
(a) displaying (S105), on display unit (111) of the eyesight examination device (100), an optotype image selected from a plurality of optotype image groups and four reference images located on the left side, the right side, the lower side and the upper side of the optotype image; wherein each of the optotype image groups includes different optotype images for identical shapes;
(b) receiving (S107) a direction input indicating the direction from the displayed optotype image to one reference image of the displayed four reference images, wherein the direction input is generated through the direction stick of the eyesight examination device (100) for selecting 4-direction; and
(c) determining (S109), by the control unit (117) of the eyesight examination device (100), whether the reference image corresponding to the direction input among the four reference images has an identical shape with that of the optotype image, wherein
one of the four reference images has the identical shape with that of the optotype image, and
wherein in step (a) the optotype image groups are pre-drawn on display unit (111); and wherein in step (a) the plurality of LEDs of the eyesight examination device (100) illuminate the four reference images of the reference optotype plate (111-3) of the eyesight examination device (100).

2. The eyesight examination method according to claim 1, wherein
the size of the reference images is equal to or greater than the size of the largest one of optotype images of the optotype image group having the same shape.

3. The eyesight examination method according to claim 1, further comprising step (d) of displaying a subsequent optotype image selected according to the determination result of step (c) and four reference images located on a left side, a right side, a lower side and an upper side from the subsequent optotype image,
wherein in steps (a) and (d), for binocular examination of a subject, two optotype images are displayed and two sets of the four reference images are displayed to be positioned outside on a left side, a right side, a lower side and an upper side of each of the optotype images, respectively.

4. An eyesight examination device (100), comprising:
a display unit (111) including one or two display modules (111-1) and a reference optotype plate (111-3) configured to display an optotype image selected from a plurality of optotype image groups and four reference images located on a left side, a right side, a lower side and an upper side from the optotype image;
an illumination unit (113) including a plurality of LEDs configured to illuminate the four reference images of the reference optotype plate (111-3);
a direction stick for selecting 4-direction; and
a control unit (117) configured to receive a direction input generated through the direction stick and to determine whether the reference image corresponding to the direction input among the four reference images has the identical shape with that of the optotype image,
wherein
one of the four reference images has the identical shape with that of the optotype image, and
wherein the optotype image groups are pre-drawn on the display unit (111); and
wherein the four reference images of the reference optotype plate (111-3) are illuminated by the plurality of LEDs during the eyesight examination.

5. The eyesight examination device according to claim 4, wherein
the control unit selects a subsequent optotype image according to the determination result of the corresponding reference image and the optotype image and outputs the selected subsequent optotype image and the four reference images located on a left side, a right side, a lower side and an upper side from the subsequent optotype image through the display unit, and
for binocular examination of a subject, the display unit displays two optotype images and two sets of the four reference images positioned outside on a left side, a right side, a lower side and an upper side of each of the optotype images respectively.

6. The eyesight examination device according to claim 5, wherein the display unit includes two display modules for displaying the optotype images respectively and the reference optotype plate accommodates the two display modules.

7. A downloader server configured to store programs of an eyesight examination method, comprising:
a communication module configured to receive a download request through a network and transmitting a program corresponding to the download request through the network;
a storage module, having stored eyesight examination programs which, when executed on the eyesight examination device (100) according to claim 4, perform the eyesight examination method of claim 1; and
a control module configured to transfer the eyesight examination program stored in the storage module to the communication module according to the download request for the eyesight examination program, which is received through the communication module.

## Patentansprüche

1. Sehtestverfahren zur Durchführung mit einer Sehtestvorrichtung (100) gemäß Anspruch 4, umfassend die Schritte:
(a) Anzeigen (S105) eines Sehzeichenbilds, das aus einer Vielzahl von Sehzeichenbildergruppen ausgewählt ist, und von vier Referenzbildern, die sich auf der linken Seite, der rechten Seite, der unteren Seite und der oberen Seite des Sehzeichenbilds befinden, auf einer Anzeigeeinheit (111) der Sehtestvorrichtung (100); wobei jede der Sehzeichenbildergruppen verschiedene Sehzeichenbilder für identische Formen umfasst;
(b) Empfangen (S107) einer Richtungseingabe, die die Richtung von dem angezeigten Sehzeichenbild zu einem Referenzbild der vier angezeigten Referenzbilder angibt, wobei die Richtungseingabe durch den Richtungsstick der Sehtestvorrichtung (100) zum Auswählen einer 4-Richtung erzeugt wird; und
(c) Bestimmen (S109) durch die Steuerungseinheit (117) der Sehtestvorrichtung (100), ob das Referenzbild, das der Richtungseingabe entspricht, unter den vier Referenzbildern dieselbe Form hat wie das Sehzeichenbild, wobei
eines der vier Referenzbilder dieselbe Form wie das Sehzeichenbild aufweist und
wobei in Schritt (a) die Sehzeichenbildergruppen auf der Anzeigeeinheit (111) vorgezeichnet sind; und wobei in Schritt (a) die Vielzahl von LEDs der Sehtestvorrichtung (100) die vier Referenzbilder der Referenzsehzeichenplatte (111-3) der Sehtestvorrichtung (100) beleuchten.

2. Sehtestverfahren gemäß Anspruch 1, wobei
die Größe der Referenzbilder größer oder gleich der Größe des größten der Sehzeichenbilder der Sehzeichenbildergruppe mit derselben Form ist.

3. Sehtestverfahren gemäß Anspruch 1, weiterhin umfassend Schritt (d), bei dem ein anschließendes Sehzeichenbild, das gemäß dem Bestimmungsergebnis von Schritt (c) ausgewählt ist, und vier Referenzbilder, die sich auf der linken Seite, der rechten Seite, der unteren Seite und der oberen Seite des anschließenden Sehzeichenbilds befinden, angezeigt werden,
wobei in den Schritten (a) und (d) für die binokulare Untersuchung eines Probanden zwei Sehzeichenbilder angezeigt werden und zwei Gruppen der vier Referenzbilder so angezeigt werden, dass sie sich außerhalb auf der linken Seite, der rechten Seite, der unteren Seite bzw. der oberen Seite jedes der Sehzeichenbilder befinden.

4. Sehtestvorrichtung (100), umfassend:
eine Anzeigeeinheit (111), die ein oder zwei Anzeigemodule (111-1) und eine Referenzsehzeichenplatte (111-3) umfasst, die so konfiguriert ist, dass sie ein aus einer Vielzahl von Sehzeichenbildergruppen ausgewähltes Sehzeichenbild und, vier Referenzbilder, die sich auf der linken Seite, der rechten Seite, der unteren Seite und der oberen Seite des Sehzeichenbilds befinden, anzeigen kann;
eine Beleuchtungseinheit (113), die eine Vielzahl von LEDs umfasst, die so konfiguriert sind, dass sie die vier Referenzbilder der Referenzsehzeichenplatte (111-3) beleuchten können;
einen Richtungsstick zum Auswählen einer 4-Richtung; und
eine Steuerungseinheit (117), die so konfiguriert ist, dass sie eine durch den Richtungsstick erzeugte Richtungseingabe empfangen kann und bestimmen kann, ob das Referenzbild, das der Richtungseingabe entspricht, unter den vier Referenzbildern dieselbe Form hat wie das Sehzeichenbild,
wobei
eines der vier Referenzbilder dieselbe Form wie das Sehzeichenbild aufweist und
wobei die Sehzeichenbildergruppen auf der Anzeigeeinheit (111) vorgezeichnet sind; und wobei die vier Referenzbilder der Referenzsehzeichenplatte (111-3) während des Sehtests durch die Vielzahl von LEDs beleuchtet werden.

5. Sehtestvorrichtung gemäß Anspruch 4, wobei
die Steuerungseinheit ein anschließendes Sehzeichenbild gemäß dem Bestimmungsergebnis des entsprechenden Referenzbilds und dem Sehzeichenbild auswählt und das anschließende Sehzeichenbild und die vier Referenzbilder, die sich auf der linken Seite, der rechten Seite, der unteren Seite und der oberen Seite des anschließenden Sehzeichenbilds befinden, über die Anzeigeeinheit ausgibt und
für die binokulare Untersuchung eines Probanden die Anzeigeeinheit zwei Sehzeichenbilder und zwei Gruppen der vier Referenzbilder, die sich außerhalb auf der linken Seite, der rechten Seite, der unteren Seite bzw. der oberen Seite jedes der Sehzeichenbilder befinden, anzeigt.

6. Sehtestvorrichtung gemäß Anspruch 5, wobei die Anzeigeeinheit zwei Anzeigemodule zum Anzeigen der jeweiligen Sehzeichenbilder umfasst und die Referenzsehzeichenplatte die zwei Anzeigemodule beherbergt.

7. Download-Server, der so konfiguriert ist, dass er Programme eines Sehtestverfahrens speichern kann, umfassend:
ein Kommunikationsmodul, das so konfiguriert ist, dass es eine Download-Anfrage über ein Netzwerk empfangen kann und ein Programm, das der Download-Anfrage entspricht, über das Netzwerk senden kann;
in Speichermodul, auf dem Sehtestprogramme gespeichert sind, die, wenn sie mit der Sehtestvorrichtung (100) gemäß Anspruch 4 ausgeführt werden, das Sehtestverfahren gemäß Anspruch 1 durchführen; und
ein Steuerungsmodul, das so konfiguriert ist, dass es das im Speichermodul gespeicherte Sehtestprogramm gemäß der Download-Anfrage für das Sehtestprogramm, die über das Kommunikationsmodul empfangen wird, auf das Kommunikationsmodul übertragen kann.

## Revendications

1. Procédé pour test de vision, à effectuer dans un dispositif pour test de vision (100) selon la revendication 4, comprenant les étapes consistant à
(a) afficher (S105), sur l'unité d'affichage (111) du dispositif pour test de vision (100), une image d'optotype choisie dans une pluralité de groupes d'images d'optotype, et quatre images de référence situées sur le côté gauche, le côté droit, le côté inférieur et le côté supérieur de l'image d'optotype, dans lequel chacun des groupes d'images d'optotype comprend des images d'optotype différentes pour des formes identiques,
(b) recevoir (S107) un intrant de direction indiquant la direction de l'image d'octotype affichée vers une image de référence parmi les quatre images de référence affichées, dans lequel ledit intrant de direction est généré au moyen du stick directionnel dudit dispositif pour test de vision (100) pour choisir une 4-direction, et
(c) déterminer (S109), par l'unité de commande (117) dudit dispositif pour test de vision (100), si l'image de référence correspondant au intrant de direction parmi les quatre images de référence présente une forme identique à celle de l'image d'optotype, où
l'une parmi les quatre images de référence présente une forme identique à celle de l'image d'optotype, et
dans lequel les groupes d'images d'optotype dans l'étape (a) sont tracés sur l'unité d'affichage (111), et dans lequel la pluralité des LEDs dudit dispositif pour test de vision (100) dans l'étape (a) éclairent les quatre images de référence de la plaque d'optotypes de référence (111-3) dudit dispositif pour test de vision (100).

2. Procédé pour test de vision selon la revendication 1, dans lequel
la taille des images de référence est supérieure ou égale à la taille de la plus grande des images d'optotype du groupe d'images d'optotype ayant la même forme.

3. Procédé pour test de vision selon la revendication 1, comprenant en outre l'étape (d) consistant à afficher une image d'optotype suivante choisie selon le résultat de détermination de l'étape (c), et quatre images de référence situées sur le côté gauche, le côté droit, le côté inférieur et le côté supérieur de l'image d'optotype suivante,
dans lequel dans les étapes (a) et (d), pour l'examen binoculaire d'un patient, deux images d'optotype sont affichées, et deux ensembles des quatre images de référence sont affichés pour être situés sur le côté gauche, le côté droit, le côté inférieur et le côté supérieur de chacune des images d'optotype, respectivement.

4. Dispositif pour test de vision (100), comprenant :
une unité d'affichage (111) comprenant un ou deux modules d'affichage (111-1), et une plaque d'optotypes de référence (111-3) configurée pour afficher une image d'optotype choisie dans une pluralité de groupes d'images d'optotype, et quatre images de référence situées sur le côté gauche, le côté droit, le côté inférieur et le côté supérieur de l'image d'optotype,
une unité d'éclairage (113) comprenant une pluralité de LEDs configurées pour éclairer les quatre images de référence de la plaque d'optotypes de référence (111-3),
un stick directionnel pour choisir la 4-direction, et
une unité de commande (117) configurée pour recevoir un intrant de direction généré au moyen du stick directionnel, et pour déterminer si l'image de référence correspondant au intrant de direction parmi les quatre images de référence présente une forme identique à celle de l'image d'optotype,
où
l'une parmi les quatre images de référence présente une forme identique à celle de l'image d'optotype, et
dans lequel les groupes d'images d'optotype sont tracés sur l'unité d'affichage (111), et dans lequel les quatre images de référence de la plaque d'optotypes de référence (111-3) sont éclairées par la pluralité des LEDs pendant le test de vision.

5. Dispositif pour test de vision selon la revendication 4, dans lequel
ladite unité de commande choisit une image d'optotype suivante selon le résultat de détermination de l'image de référence correspondante et l'image d'optotype, et sort l'image d'optotype suivante choisie et les quatre images de référence situées sur le côté gauche, le côté droit, le côté inférieur et le côté supérieur de l'image d'optotype suivante à travers l'unité d'affichage, et
pour l'examen binoculaire d'un patient, ladite unité d'affichage affiche deux images d'optotype et deux ensembles des quatre images de référence situées sur le côté gauche, le côté droit, le côté inférieur et le côté supérieur de chacune des images d'optotype, respectivement.

6. Dispositif pour test de vision selon la revendication 5, dans lequel ladite unité d'affichage comprend deux modules d'affichage pour afficher les images d'optotype, respectivement, et la plaque d'optotypes de référence accueille les deux modules d'affichage.

7. Serveur de téléchargement, configuré pour enregistrer des programmes d'un procédé pour test de vision, comprenant :
un module de communication configuré pour recevoir une demande de téléchargement à travers un réseau, et transmettre un programme correspondant à la demande de téléchargement à travers le réseau,
un module de stockage, sur lequel sont stockés des programmes de test de vision, qui, lorsqu'ils sont exécutés sur le dispositif pour test de vision (100) selon la revendication 4, effectuent le procédé pour test de vision selon la revendication 1, et
un module de commande configuré pour transférer le programme de test de vision enregistré dans le module de stockage au module de communication selon la demande de téléchargement pour le programme de test de vision, qui est reçu à travers le module de communication.
